# FASCICULE DE BREVET EUROPEEN

(11) **EP 0 625 972 B1**
(45) Date de publication et mention de la délivrance du brevet: **13.03.1996**
(21) Numéro de dépôt: 93902312.3
(22) Date de dépôt: 09.12.1992
(51) Int. Cl.: C07D 277/82

(54) **PROCEDE DE PREPARATION D'AMINO-2 NITRO-7 BENZOTHIAZOLES**
VERFAHREN ZUR HERSTELLUNG VON 2-AMINO-7-NITROBENZOTHIAZOLEN
PROCESS FOR THE PREPARATION OF 2-AMINO-7-NITROBENZOTHIAZOLES

(30) Priorité: 13.12.1991 FR 9115486
(43) Date de publication de la demande: 30.11.1994
(73) Titulaire: RHONE-POULENC RORER S.A., F-92160 Antony (FR)
(72) Inventeur: AUDIAU, François, F-94220 Charenton-le-Pont (FR); JIMONET, Patrick, F-78450 Villepreux (FR); MIGNANI, Serge, F-94290 Chatenay Malabry (FR)
(74) Mandataire: Savina, Jacques
(86) Numéro de dépôt international: FR9201165
(87) Numéro de publication internationale: WO9312099

(56) Documents cités:
- EP-A- 0 374 041
- CHEMICAL ABSTRACTS, vol. 41, 1947, Columbus, Ohio, US; abstract no. 754c,
- CHEMICAL & PHARMACEUTICAL BULLETIN vol. 27, no. 1, 1979, pages 1 - 11 N.SUZUKI ET AL
- JOURNAL OF THE AMERICAN CHEMICAL SOCIETY vol. 73, no. 8, 1951, pages 4007 - 4010 L. KATZ

## Description

La présente invention concerne un procédé de préparation d'amino-2 nitro-7 benzothiazoles de formule : 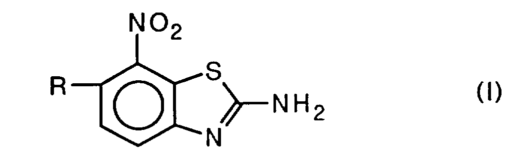 dans laquelle R représente un radical polyfluoroalcoxy.

La nitration directe d'amino-2 benzothiazoles substitués en position -6 conduit à un mélange d'amino-2 nitro-4 et nitro-5 benzothiazoles (EP 374041).

Il est également connu de préparer les nitro-4, -5, -6 ou -7 amino-2 benzothiazoles par réaction de dinitrochlorobenzènes ou dinitrophényl thiocyanates avec la thiourée ou le thiocyanate d'ammonium (brevet US 4808723, R. HAMPRECH, Chem. Abstr., 101, 211131 et J. SCHULZE et coll., Z. Chem., 20, 436 (1980)); par réaction de nitrophénylthiourées avec S₂Cl₂ ou un complexe plomb(lV)-phosphate (S. CLAUDE et coll., Helv. Chim. Acta, 64, 1545 (1981); S.A. Von MAHMOUD et coll., Prakt. Chemie, 316, 154 (1974). Cependant, ces cyclisations ne sont pas toujours régiosélectives.

Il a maintenant été trouvé et c'est ce qui fait l'objet de la présente demande, un procédé permettant d'obtenir industriellement les composés de formule (I) avec de bons rendements.

Ce procédé consiste à
a) nitrer un dérivé de formule : 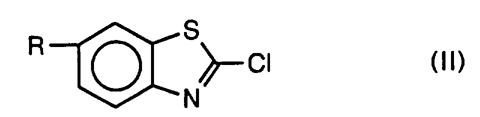 dans laquelle R a les mêmes significations que dans la formule (I), et
b) faire réagir le composé de formule : 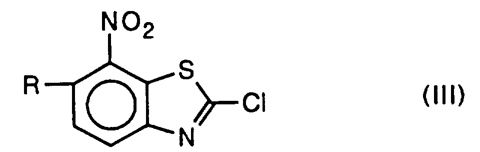 dans laquelle R a les mêmes significations que dans la formule (I), ainsi obtenu avec l'hydroxyde d'ammonium.

Il est particulièrement avantageux d'effectuer la nitration de l'étape a) au moyen d'un mélange acide nitrique-acide sulfurique.

Généralement, on utilise un mélange acide nitrique-acide sulfurique comprenant 1 partie en volume d'acide nitrique concentré et 1 à 5 parties en poids d'acide sulfurique concentré. Il est préférable d'utiliser un mélange comprenant 1 partie d'acide nitrique concentré et 3,7 parties d'acide sulfurique concentré.

La quantité du mélange acide nitrique-acide sulfurique est comprise entre 1 et 10 parties en volume pour une partie en poids du dérivé de formule (Il) mis en oeuvre.

La température réactionnelle est de préférence comprise entre 0°C et 20°C.

L'étape b) s'effectue généralement au sein d'un solvant organique inerte tel qu'un alcool (méthanol, éthanol par exemple), dans un réacteur fermé.

Il est particulièrement avantageux d'opérer à une température comprise entre 10°C et 150°C.

On utilise généralement 5 à 20 parties en poids d'hydroxyde d'ammonium pour une partie en poids du dérivé de formule (III) mis en oeuvre.

Les dérivés de formule (Il) peuvent être obtenus par application ou adaptation de la méthode décrite dans le brevet EP 43013.

Les composés de formule (I) peuvent être séparés du mélange réactionnel selon les techniques habituelles de séparation (extraction, chromatographie, cristallisation...).

Les composés de formule (I) sont utiles comme médicaments ou intermédiaires pour préparer des médicaments (EP 282971 et 374041) ou comme intermédiaires pour colorants (brevets US 2149051, 4363913, GB 2163174).

L'exemple suivant montre comment l'invention peut être mise en oeuvre.

### EXEMPLE 1

### a) Préparation du chloro-2 nitro-7 trifluorométhoxy-6 benzothiazole

10 g de chloro-2 trifluorométhoxy-6 benzothiazole sont ajoutés, goutte à goutte, en une heure, à un mélange refroidi à 10°C d'acide sulfurique concentré (50 cm3) et d'acide nitrique concentré (25 cm3). Le mélange est ensuite chauffé une heure à 60°C. Après refroidissement, le mélange est versé dans un mélange eau-glace (1/1 en poids) et extrait avec 3 fois 100 cm3 de chloroforme. Les phases organiques sont séchées sur sulfate de magnésium et concentrées sous vide (2,7 kPa). Le résidu est purifié par flash-chromatographie avec un mélange chloroforme-cyclohexane (4/6 en volumes) comme éluant. On obtient ainsi 6,4 g de chloro-2 nitro-7 trifluorométhoxy-6 benzothiazole fondant à 64°C.

### b) Préparation de l'amino-2 nitro-7 trifluorométhoxy-6 benzothiazole

Un mélange de 0,4 g de chloro-2 nitro-7 trifluorométhoxy-6 benzothiazole et 10 cm3 d'une solution aqueuse à 33% d'hydroxyde d'ammonium dans 30 cm3 d'éthanol est chauffé à 110°C dans un autoclave pendant 5 heures. Le mélange est refroidi et le solvant est évaporé sous pression réduite (2,7 kPa). Après purification par flash-chromatographie sur silice avec un mélange cyclohexane-acétate d'éthyle (4/6 en volumes) comme éluant, on obtient 0,22 g d'amino-2 nitro-7 trifluorométhoxy-6 benzothiazole fondant à 180°C.

### c) Préparation des intermédiaires

49,4 g d'hydrazino-2 trifluorométhoxy-6 benzothiazole sont ajoutés en une heure à 97,9 g de chlorure de thionyle chauffé à 50°C. Le mélange est ensuite chauffé une heure à cette même température puis refroidi à 0°C. Après addition de 200 g d'un mélange eau-glace (1/1 en poids), le précipité est filtré et lavé avec 2 fois 50 cm3 d'eau. On obtient ainsi 54,5 g de chloro-2 trifluorométhoxy-6 benzothiazole fondant à 50°C.

A une suspension de 93,6 g d'amino-2 trifluorométhoxy-6 benzothiazole dans 420 cm3 d'éthylène glycol sous courant d'azote, on ajoute 48 g d'hydrazine hydratée et 42 g de dichlorhydrate d'hydrazine. Le mélange est chauffé 2 heures à 140°C. Après refroidissement, le précipité est filtré et trituré avec un mélange eau-éther diéthylique (1/1 en volumes). On obtient ainsi 89,9 g d'hydrazino-2 trifluorométhoxy-6 benzothiazole fondant à 208°C.

L'amino-2 trifluorométhoxy-6 benzothiazole peut être préparé selon la méthode décrite par L.M. YAGUPOLSKII et coll., Zh. Obshch. Khim., 33 (7), 2301 (1963).

## Revendications

1. Procédé de préparation des composés de formule : 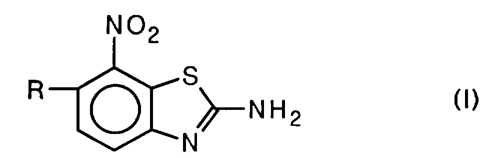 dans laquelle R représente un radical polyfluoroalcoxy caractérisé en ce que
a) on nitre un dérivé de formule : 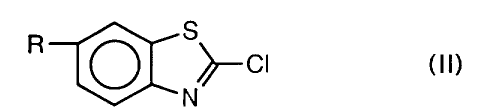 dans laquelle R a les mêmes significations que dans la formule (I), et
b) on fait réagir le composé de formule : 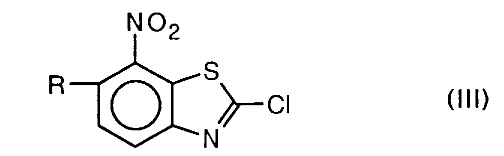 dans laquelle R a les mêmes significations que dans la formule (I), ainsi obtenu avec l'hydroxyde d'ammonium.

2. Procédé selon la revendication 1 caractérisé en ce que l'on effectue la nitration au moyen d'un mélange acide nitrique-acide sulfurique.

3. Procédé selon l'une des revendications 1 ou 2 caractérisé en ce que l'on utilise un mélange acide nitrique-acide sulfurique comprenant 1 partie en volume d'acide nitrique concentré et 1 à 5 parties en poids d'acide sulfurique concentré.

4. Procédé selon l'une des revendications 1 à 3 caractérisé en ce que l'on utilise 1 à 10 parties en volume du mélange acide nitrique-acide sulfurique pour une partie en poids du composé de formule (Il) mis en oeuvre.

5. Procédé selon l'une des revendications 1 à 4 caractérisé en ce que l'on effectue la nitration à une température comprise entre 0°C et 20°C.

6. Procédé selon l'une des revendications 1 à 5 caractérisé en ce que l'on effectue la réaction de l'étape b) dans un réacteur fermé.

7. Procédé selon l'une des revendications 1 à 6 caractérisé en ce que l'on effectue la réaction de l'étape b) au sein d'un solvant organique tel qu'un alcool.

8. Procédé selon l'une des revendications 1 à 7 caractérisé en ce que l'on utilise 5 à 20 parties en poids d'hydroxyde d'ammonium pour une partie en poids d'un dérivé de formule (III).

9. Procédé selon l'une des revendications 1 à 8 caractérisé en ce que l'on effectue la réaction de l'étape b) à une température comprise entre 10°C et 150°C.

## Patentansprüche

1. Verfahren zur Herstellung von Verbindungen der Formel (I), 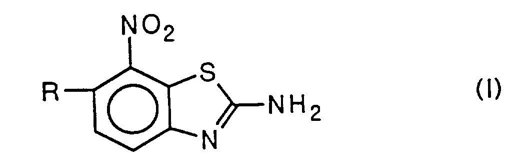 worin R einen Polyfluoroalkoxyrest bedeutet, dadurch gekennzeichnet, daß
a) man ein Derivat der Formel (II) 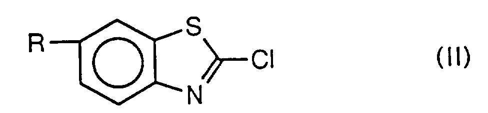 worin R die gleichen Bedeutungen wie in Formel (I) hat, nitriert und
b) daß man die so erhaltene Verbindung der Formel (III) 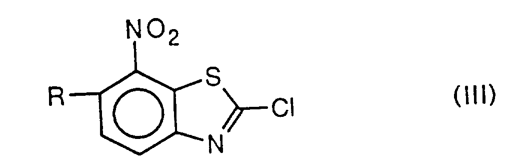 worin R die gleichen Bedeutungen wie in Formel (I) hat, mit Ammoniumhydroxid zur Reaktion bringt.

2. Verfahren gemäß Anspruch 1, dadurch gekennzeichnet, daß man die Nitrierung mit einem Gemisch von Salpetersäure-Schwefelsäure durchführt.

3. Verfahren gemäß einem der Ansprüche 1 oder 2, dadurch gekennzeichnet, daß man ein Gemisch Salpetersäure-Schwefelsäure, das ein Volumteil konzentrierte Salpetersäure und 1 bis 5 Gewichtsteile konzentrierte Schwefelsäure enthält, verwendet.

4. Verfahren gemäß einem der Anspüche 1 bis 3, dadurch gekennzeichnet, daß man 1 bis 10 Volumteile des Gemisches Salpetersäure-Schwefelsäure für einen Gewichtsteil der eingesetzten Verbindung der Formel (II) verwendet.

5. Verfahren gemäß einem der Ansprüche 1 bis 4, dadurch gekennzeichnet, daß man die Nitrierung bei einer Temperatur zwischen 0°C und 20°C durchführt.

6. Verfahren gemäß einem der Anspüche 1 bis 5, dadurch gekennzeichnet, daß man die Reaktion der Stufe b) in einem geschlossenen Reaktionsgefäß durchführt.

7. Verfahren gemäß einem der Ansprüche 1 bis 6, dadurch gekennzeichnet, daß man die Reaktion der Stufe b) in einem organischen Lösungsmittel, wie einem Alkohol bewirkt.

8. Verfahren gemäß einem der Ansprüche 1 bis 7, dadurch gekennzeichnet, daß man 5 bis 20 Gewichtsteile Ammoniumhydroxid für einen Gewichtsteil eines Derivats der Formel (III) verwendet.

9. Verfahren gemäß einem der Ansprüche 1 bis 8, dadurch gekennzeichnet, daß man die Reaktion der Stufe b) bei einer Temperatur zwischen 10°C und 150°C durchführt.

## Claims

1. Process for the preparation of the compounds of formula: 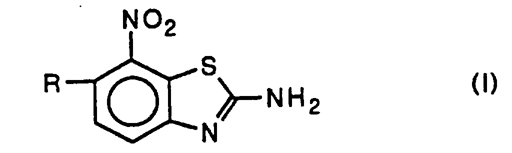 in which R represents a polyfluoroalkoxy radical, characterized in that
a) a derivative of formula: 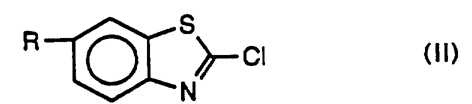 in which R has the same meanings as in formula (I) is nitrated, and
b) the compound of formula: 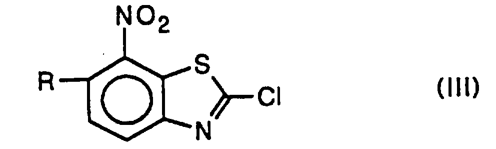 in which R has the same meanings as in formula (I), thus obtained is reacted with ammonium hydroxide.

2. Process according to claim 1, characterized in that the nitration is carried out using a nitric acid/sulphuric acid mixture.

3. Process according to either of claims 1 and 2, characterized in that a nitric acid/sulphuric acid mixture is used containing 1 part by volume of concentrated nitric acid and 1 to 5 parts by weight of concentrated sulphuric acid.

4. Process according to one of claims 1 to 3, characterized in that 1 to 10 parts by volume of nitric acid/sulphuric acid mixture are used per one part by weight of the compound of formula (II) used.

5. Process according to one of claims 1 to 4, characterized in that the nitration is carried out at a temperature between 0°C and 20°C.

6. Process according to one of claims 1 to 5, characterized in that the reaction of step b) is carried out in a closed reactor.

7. Process according to one of claims 1 to 6, characterized in that the reaction of step b) is carried out in an organic solvent such as an alcohol.

8. Process according to one of claims 1 to 7, characterized in that 5 to 20 parts by weight of ammonium hydroxide are used per one part by weight of a derivative of formula (III).

9. Process according to one of claims 1 to 8, characterized in that the reaction of step b) is carried out at a temperature of between 10°C and 150°C.
